# EUROPEAN PATENT SPECIFICATION

(11) **EP 3 927 290 B1**
(45) Date of publication and mention of the grant of the patent: **30.11.2022**
(21) Application number: 20742514.1
(22) Date of filing: 03.02.2020
(51) Int. Cl.: A61F 5/37

(54) **SELF-WRAPPING SAFETY DEVICE FOR THE PHYSICAL RESTRAINT OF A PERSON**
SELBSTWICKELNDE SICHERHEITSVORRICHTUNG FÜR DIE KÖRPERRÜCKHALTUNG EINER PERSON
DISPOSITIF DE SÉCURITÉ À AUTO-ENVELOPPEMENT POUR LA RETENUE PHYSIQUE D'UNE PERSONNE

(30) Priority: 18.02.2019 IT 201900002283
(43) Date of publication of application: 29.12.2021
(73) Proprietor: Zaganelli, Damiano, 44123 Ferrara (FE) (IT)
(72) Inventor: Zaganelli, Damiano, 44123 Ferrara (FE) (IT)
(74) Representative: Cataldi, Silvia
(86) International application number: PCT/IT2020/050017
(87) International publication number: WO 2020/170280

(56) References cited:
- FR-A1- 2 880 259
- IT-A1- UB20 159 284
- US-A1- 2014 150 807
- US-A1- 2015 257 919

## Description

Self-wrapping safety device for the physical restraint of a person.

The present invention relates to a self-wrapping safety device for the physical restraint of a person as defined in the claims.

### Technical field

In more detail the invention relates to the structure and operation of a self-wrapping safety device for the physical restraint of a person, who shows, for example, any behaviour potentially dangerous to themselves or to others, so as to limit and control the movements of that person.

The following description will relate to physical restraint in a health-care context but it is evident that it must not be considered to be limited to that specific use.

### Background art

As is known, the expression "physical restraint", in a health-care context, means any device, tool or equipment whether fixed or movable, arranged near the body of a person which is difficult for that person to reach, manoeuvre or remove.

Typically, such devices are used, when strictly necessary, by health-care and/or public safety personnel within hospital structures, residential care homes, public places or the like, to prevent serious risks to the subject being treated or who requires temporary immobilization by external persons.

There are currently various physical restraint devices and equipment configured to limit the voluntary movements of a person.

Such devices comprise, for example, devices fixable to structures, such as a bed, a chair or a table, and devices fixable directly to the body or to a segment of the body of the patient, for example a wrist, an ankle, and so on.

ITUB20159284 A1 discloses a self-wrapping safety device for the physical restraint of a person, comprising a main body, provided with a first and a second end and configured to get in contact with a person, at least one self-wrapping strip, coupled to said main body provided with a first and a second end, said main body and said at least one self-wrapping strip being configured to each other so as to be able to move both from a coplanar, first position to a second position, in which said self-wrapping strip and said main body are arranged wrapped and rolled up on themselves or around said person.

Typically, the prior art solutions comprise, amongst other things, handcuffs, or bands and belts with two opposite ends equipped with bands of Velcro able to adhere to each other, allowing the device to wrap around the body of the patient, immobilizing them.

However, one of the main disadvantages of such prior art solutions is the fact that they do not allow rapid and effective wrapping of the body of the patient. Indeed, often stabilization of the patient by means of such devices is not optimal, therefore it does not allow care operators to easily control the movements of the patient.

Another disadvantage of such prior art solutions is the fact that they do not guarantee patient postural stability, therefore risking causing physical harm to that same patient.

A further disadvantage of such prior art solutions is the fact that they require excessive efforts by the care operators to immobilize the patient in safety conditions.

Another disadvantage of such prior art solutions is the fact that they may cause direct or indirect harm to the patient due to the pressure exerted on the body of the patient.

### Aim of the invention

The aim of the present invention is to overcome the above-mentioned disadvantages, by providing a self-wrapping safety device for the physical restraint of a person.

Another aim of the invention is to provide a self-wrapping safety device for the physical restraint of a person which allows that same person to be immobilized rapidly, effectively and is easy to use.

A further aim of the invention is to provide a self-wrapping safety device for the physical restraint of a person whose use does not cause accidental harm to that same person and to the external persons who use it.

Another aim of the invention is to provide a self-wrapping safety device for the physical restraint of a person which is highly reliable, relatively easy to make, and has competitive costs if compared with the prior art.

### Object of the invention

Therefore, the specific object of the present invention is a self-wrapping safety device for the physical restraint of a person, comprising a main body, provided with a first and a second end and configured to get in contact with a person, at least one self-wrapping strip, coupled to said main body, provided with a first and a second end, said main body and at least one self-wrapping strip being configured to each other so as to be able to move both from a first position, wherein said first and second end of said main body are arranged opposite to each other and coplanar and said first and second end of said at least one self-wrapping strip are arranged opposite to each other and coplanar, to a second position, wherein said self-wrapping strip and said main body are arranged wrapped and rolled up on themselves or around said person, said self-wrapping safety device may also comprise at least one pair of activation handles coupled to said at least one self-wrapping strip, configured to cause said at least one self-wrapping strip and therefore said main body, moving from said first position to said second position.

Furthermore according to the invention, each activation handle of said at least one pair of activation handles, in use, is subjected to a tensile force exerted by a user, causing said passage of said at least one self-wrapping strip and of said main body, from said first position to said second position.

Moreover according to the invention, each activation handle of said at least one pair of activation handles is provided with a cable for the grip of said activation handle by a user.

Preferably according to the invention, said cable comprises in its turn a first and a second end fixed to said at least one self-wrapping strip by connection means.

Furthermore according to the invention, said device may further comprise a first and a second fixing elements respectively arranged on said first and said second end of said main body, said first and said second fixing element being configured to hook to each other in said second position.

Moreover according to the invention, said device may comprise a plurality of support handles integrally fixed with said main body and configured to allow said main body to be gripped and moved both in said first position and in said second position.

Preferably according to the invention, said device may comprise a plurality of self-wrapping strips.

Furthermore according to the invention, said main body comprises a first and a second surface.

Moreover according to the invention, said self-wrapping strips are arranged within said main body, between said first and second surface of said main body.

Preferably according to the invention, said main body comprises inside it a plurality of supporting strips, having a development along the Y axis direction and arranged in correspondence of at least one self-wrapping strip.

### Brief description of the figures

The invention is now described, by way of example and without limiting the scope of the invention, with reference to the accompanying drawings which illustrate preferred embodiments of it, in which:
Figure 1 shows a perspective side view of a self-wrapping safety device for the physical restraint of a person in an open configuration, the object of the present invention;
Figure 2 shows a perspective side view of the device shown in Figure 1, in a closed configuration;
Figure 3 shows a top view of the device of Figure 1;
Figure 4 shows a side section of a first embodiment of a connection between an activation handle and a strip included in the device of Figure 1;
Figure 5 shows a side section and in detail, a fixing element for fixing the activation handle to the strip, according to Figure 4;
Figure 6a shows a front view in detail of a second embodiment of a connection between the activation handle and the strip included in the device of Figure 1;
Figure 6b shows a side view in section of the second embodiment of a connection between the activation handle and the strip shown in Figure 6a.
Figure 7a shows a perspective side view of the strip in a first position wherein it is convex in the XY plane relative to the positive Z axis;
Figure 7b shows a perspective side view of the strip in a second position wherein it is concave in the XY plane relative to the positive Z axis; and
Figure 8 shows a top view of a further embodiment of the device of Figure 1.

The similar parts will be indicated in the various drawings with the same numerical references.

### Detailed description

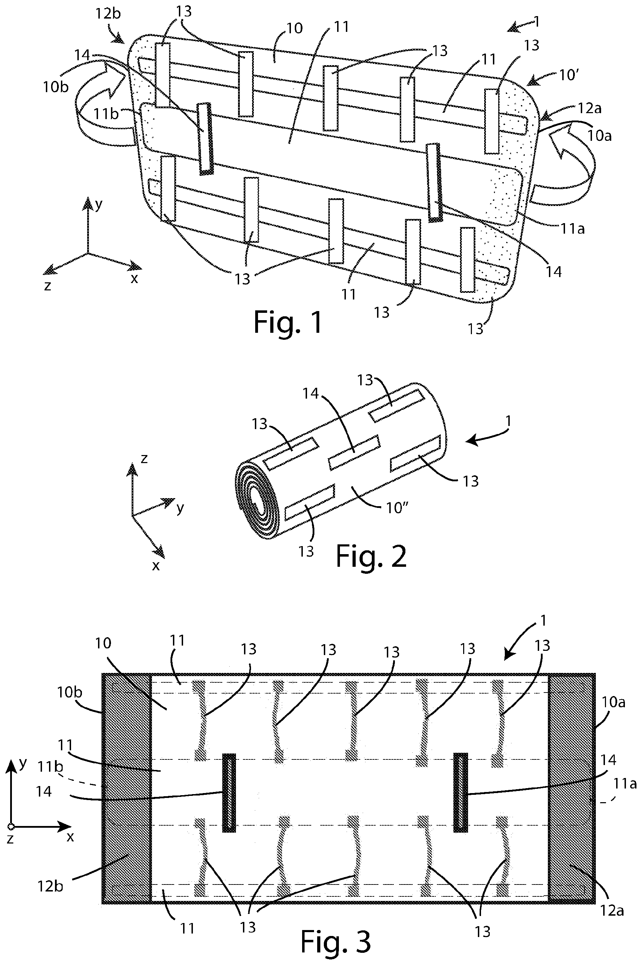

With reference to Figures 1 and 2, the self-wrapping safety device for the physical restraint of a person according to the present invention, indicated in its entirety with the reference number 1, comprises a main body 10, at least one self-wrapping strip 11 coupled to said main body 10, at least one pair of activation handles 14 coupled to said at least one self-wrapping strip 11 and a plurality of support handles 13 arranged on said main body 10.

The main body 10 of the device 1 according to the present invention is able to move from a first position to a second position wherein it is arranged wrapped and rolled up on itself or around the person who needs to be immobilized.

In particular, said main body 10 comprises a first 10ₐ and a second 10_{b} end.

Said first 10ₐ and second 10_{b} end are arranged, in said first position, opposite to each other and coplanar, whilst, in said second position, they are wrapped on themselves or on said person.

As can be seen in Figures 1 and 2, in the embodiment described, said main body 10 has a longitudinal development along a plane XY when it is in said first position, whilst it has a substantially cylindrical shape along the Y axis direction, when it is in said second position.

In particular, said main body 10 moves from said first position to said second position by a wrapping or rolling around the Y axis direction, in the direction indicated by the arrows shown in Figure 1.

Said main body 10 comprises a first 10' and a second 10" surface, preferably made of a flexible, strong and washable fabric.

In more detail, said first surface 10' or inner surface 10' is preferably made of a bullet-proof fabric, for example a fabric of the Kevlar type, and/or a fabric which protects against sharp weapons.

Without departing from the protective scope of the present invention, it is also possible for it to have only a central portion of said first surface 10' made of that bullet-proof fabric so as to protect in particular a respective part of the body of the user of said safety device 1. That allows, among other things, a reduction in the costs of production of said safety device 1.

Said first surface 10' is configured to protect the person immobilized from any impacts or scuffles which could cause physical harm to that same person or to the user of said device 1.

Similarly to said first surface 10', said second surface 10", or outer surface 10", is also preferably made of flexible, strong and washable material.

Said main body 10 further comprises a first 12ₐ and a second 12_{b} fixing elements respectively arranged on said second surface 10" in correspondence of said first 10ₐ and second 10_{b} end of said main body 10.

Said first 12ₐ and second 12_{b} fixing elements, made for example of Velcro bands, are configured to hook to each other when said main body 10 is in said second position.

That allows said first 10ₐ and said second 10_{b} end of said main body 10 to be fixed to each other, preventing the unintentional reopening of said safety device 1.

As can be seen in particular from Figures 1 and 3, said safety device 1 comprises at least one self-wrapping strip 11 of the known type with longitudinal development along the X axis direction.

Said self-wrapping strip 11 is preferably made of a metallic material or the like.

Said self-wrapping strip 11 may also be made of flexible plastic alloys.

Said self-wrapping strip 11 is coupled to said main body 10, and therefore, similarly to said main body 10, it is able to move from said first position to said second position.

In the embodiment which is the object of the present invention, said self-wrapping strip 11 is inserted within said main body 10, between said first 10' and second 10" surface.

Without departing from the protective scope of the present invention, it is also possible to provide other ways of coupling between said self-wrapping strip 11 and said main body 10.

For example, said self-wrapping strip 11 may be coupled to said first surface 10' or to said second surface 10".

In particular, said self-wrapping strip 11 comprises a first 11ₐ and a second 11_{b} end.

Said first 11ₐ and second 11_{b} end of each self-wrapping strip 11 are arranged, in said first position, opposite to each other and coplanar, whilst, in said second position, they are wrapped on themselves or on said person.

Said first end 11ₐ of said self-wrapping strip 11 is arranged in correspondence of said first end 10ₐ of said main body 10 and said second end 11_{b} of said self-wrapping strip 11 is arranged in correspondence of said second end 10_{b} of said main body 10.

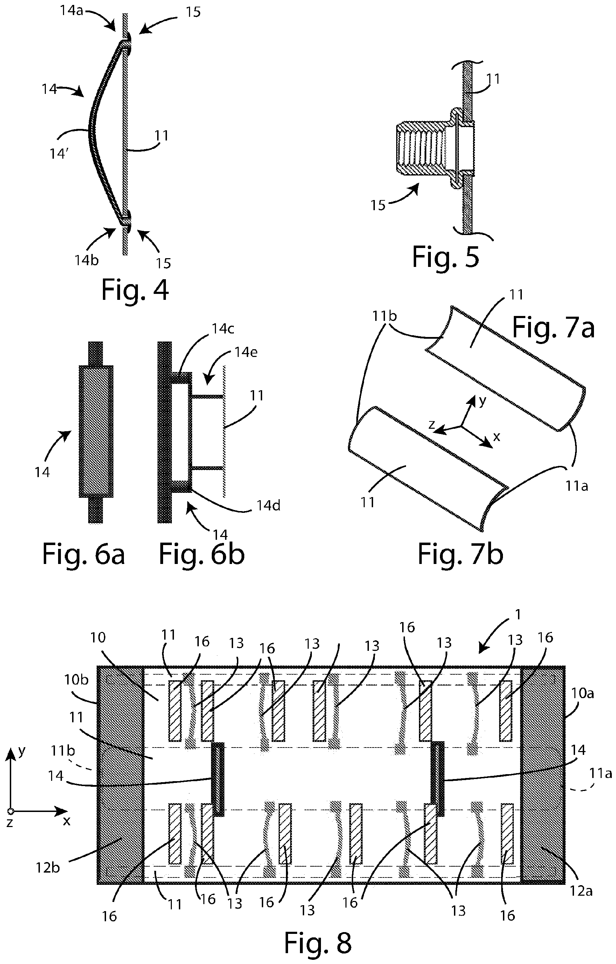

In more detail, in particular with reference to Figures 7a and 7b, each self-wrapping strip 11 is able to move from said first position wherein it is convex in the plane XY, relative to the positive Z axis, to said second position wherein it is concave, in the plane XY, relative to the same positive Z axis.

Indeed, each self-wrapping strip 11, during the movement from said first position to said second position, inverts its concavity and wraps on itself or on the person placed in contact with said main body 10.

In particular, each self-wrapping strip 11 is able to invert its concavity following a pressure exerted by the body of the person to be immobilized, or following a tensile force exerted from the outside by a user, or following a combination of such stresses.

In particular with reference to Figure 1, in the embodiment described, if the person subjected to being immobilized exerts, by means of their body, a pressure on the main body 10, and therefore on said self-wrapping strip 11, along the plane XY and towards the positive Z axis, that force will allow the inversion of concavity and therefore for wrapping of said self-wrapping strip 11 on itself.

In more detail, that force allows said self-wrapping strip 11 to move from said first position to said second position wherein it is wrapped on itself or on the person, that is to say, to invert its concavity and consequently, said main body 10 will also move from said first position to said second position, forced by the movement in the position of said self-wrapping strip 11.

As indicated, that safety device 1 comprises at least one pair of activation handles 14.

Said at least one pair of activation handles 14 is coupled to said at least one self-wrapping strip 11.

As indicated, if a user exerts a tensile force on said at least one pair of activation handles 14 towards the positive Z axis, said at least one self-wrapping strip 11, being coupled with said at least one pair of activation handles 14, will wrap on itself or on the person placed in contact with said main body 10.

In more detail, that tensile force exerted on said at least one pair of activation handles 14 allows the self-wrapping strip 11, coupled with said at least one pair of activation handles 14, to move from said first position to said second position wherein it is wrapped on itself or on the person, that is to say, to invert its concavity.

In particular with reference to Figures 4 and 5, each activation handle 14 is fixed to said at least one self-wrapping strip 11 by fixing means 15. Said fixing means 15 are for example, screws, bolts, rivets or the like.

In particular, each activation handle 14 comprises a respective cable 14', preferably made of steel, provided with a first 14ₐ and a second 14_{b} end connected to respective fixing means 15.

Suitably, that cable 14' allows an increase in the hold and resistance of said activation handle 14 in the case of stresses, for example tensile or twisting, exerted by a user.

By way of example, each activation handle 14 is preferably made of woven polymeric threads with turned-up edges to contain said cable 14' and prevent any physical harm to the user.

As can be seen from Figure 5, said fixing means 15 allow the coupling of each activation handle 14 of at least one pair of activation handles 14 with a respective self-wrapping strip 11. In that way, the user is able to activate the safety device 1 when desired.

In addition, as previously said, the combination of a tensile force exerted by a user on each activation handle 14 and of a pressure exerted by the body of the person who needs to be immobilized on said self-wrapping strip 11, allows wrapping of the self-wrapping strip 11 and therefore of the main body 10 which is faster than in the case of the individual stresses.

In order to make the safety device 1 more reactive, it is possible to insert a plurality of self-wrapping strips 11 within said main body 10, each provided with a pair of activation handles 14.

In a further embodiment of the safety device 1, said plurality of self-wrapping strip 11 comprises a first self-wrapping strip 11, or main self-wrapping strip 11, arranged in the central portion of said main body 10 and coupled to said at least one pair of activation handles 14, and at least one second self-wrapping strip 11, or secondary self-wrapping strip 11, arranged parallel to said first self-wrapping strip 11 and having a smaller size than said first self-wrapping strip 11.

If a user exerts a tensile force on said at least one pair of activation handles 14 towards the positive Z axis, said first self-wrapping strip 11, being coupled with said at least one pair of activation handles 14, is wrapped on itself or on the person placed in contact with said main body 10. Moreover, said at least one second self-wrapping strip 11, having a resistance lower than said first self-wrapping strip 11, wraps on itself or on the person placed in contact with said main body 10, at the same moment wherein wrapping of said first self-wrapping strip 11 occurs, without the use of dedicated activation handles.

Said device 1 also comprises a plurality of support handles 13.

Each support handle 13, is integrally fixed with said second surface 10" of said main body 10.

Each support handle 13 allows, for example, a user to easily grasp said safety device 1 for transporting it if necessary when it is in the closed position, or for stabilizing and controlling the movements of the person wrapped by that device 1, or the like.

Figures 6a and 6b show a second embodiment of the connection between said activation handle 14 and said self-wrapping strip 11.

Each activation handle 14 is fixed to said self-wrapping strip 11 by a connection element 14ₑ.

In particular, as can be seen from Figures 6a and 6b, each activation handle 14 is provided with a first 14_{c} and a second 14_{d} pin connected to said connection element 14ₑ.

In particular with reference to Figure 8, in a further embodiment of the present invention, said main body 10 comprises within it a plurality of supporting strips 16, between said first 10' and second 10" surface.

In particular, as can be seen from Figure 8, each supporting strip 16 has a development along the Y axis direction and is arranged in correspondence of at least one self-wrapping strip 11. In particular, each supporting strip 16 does not interfere with said at least one self-wrapping strip 11.

In more detail, each supporting strip 16 of said plurality of supporting strips 16 is configured to support, in use, said main body 10. Therefore, each supporting strip 16 is preferably made of rigid material such as, for example, plastic material or the like.

### Advantages

A first advantage of the device according to the present invention is that of minimizing the risk of physical harm when said device is applied to the body of the patient and is in use.

A further advantage of the device according to the present invention is that of guaranteeing the safety of the person to whom it is applied and of the persons close by.

Another advantage of the device according to the present invention is that of immobilizing the patient in a rapid and effective way.

A further advantage of the present invention is that of guaranteeing a rapid and effective removal of the device for assisting the patient in emergencies such as, for example, a cardiac arrest or the like.

The present invention has been described by way of example, but without limiting the scope of the invention, according to the preferred embodiments of it, but it shall be understood that it may be modified and/or adapted by experts in the field without thereby departing from the related protective scope, as defined by the appended claims.

## Claims

1. Self-wrapping safety device (1) for the physical restraint of a person, comprising:
a main body (10), provided with a first (10ₐ) and a second (10_{b}) end and configured to get in contact with a person,
at least one self-wrapping strip (11), coupled to said main body (10), provided with a first (11ₐ) and a second (11_{b}) end,
said main body (10) and said at least one self-wrapping strip (11) being configured to each other so as to be able to move both from a first position, in which said first (10ₐ) and second (10_{b}) end of said main body (10) are arranged opposite to each other and coplanar and said first (11ₐ) and second (11_{b}) end of said at least one self-wrapping strip (11) are arranged opposite to each other and coplanar, to a second position, in which said self-wrapping strip (11) and said main body (10) are arranged wrapped and rolled up on themselves or around said person,
said self-wrapping safety device (1) being **characterized in that** it comprises
at least one pair of activation handles (14) coupled to said at least one self-wrapping strip (11), configured to cause said at least one self-wrapping strip (11) and therefore said main body (10) moving from said first position to said second position.

2. Device (1) according to claim 1, **characterized in that** each activation handle (14) of said at least one pair of activation handles (14), in use, is subjected to a tensile force exerted by a user, causing said passage of said at least one self-wrapping strip (11) and of said main body (10), from said first position to said second position.

3. Device (1) according to any one of the preceding claims, **characterized in that** each activation handle (14) of said at least one pair of activation handles (14) is provided with a cable (14') for the grip of said activation handle (14) by a user.

4. Device (1) according to claim 3, **characterized in that** said cable (14') comprises in its turn a first (14ₐ) and a second (14_{b}) end fixed to said at least one self-wrapping strip (11) by connection means (15).

5. Device (1) according to any one of the preceding claims, **characterized in that** it further comprises a first (12ₐ) and a second (12_{b}) fixing elements respectively arranged on said first (10ₐ) and said second (10_{b}) end of said main body (10), said first (12ₐ) and said second (12_{b}) fixing element being configured to hook to each other in said second position.

6. Device (1) according to any one of the preceding claims, **characterized in that** it comprises a plurality of support handles (13) integrally fixed with said main body (10) and configured to allow said main body (10) to be gripped and moved both in said first position and in said second position.

7. Device (1) according to any one of the preceding claims, **characterized in that** it comprises a plurality of self-wrapping strips (11).

8. Device (1) according to any one of the preceding claims, **characterized in that** said main body (10) comprises a first (10') and a second (10") surface.

9. Device (1) according to claim 8, **characterized in that** said self-wrapping strips (11) are arranged within said main body (10), between said first (10') and second (10") surface of said main body (10).

10. Device (1) according to any one of the preceding claims, **characterized in that** said main body (10) comprises a plurality of supporting strips (16), having a development along the Y axis direction and arranged in correspondence of at least one self-wrapping strip (11).

## Patentansprüche

1. Selbstwickelndes Sicherheitsgerät (1) für das physische Festhalten einer Person, umfassend:
Einen Hauptkörper (10), der ein erstes (10ₐ) und ein zweites (10_{b}) Ende besitzt
und konfiguriert ist, um mit einer Person in Kontakt zu treten,
zumindest einen selbstwickelnden Streifen (11), der an den besagten Hauptkörper (10) angeschlossen ist,
der ein erstes (11ₐ) und ein zweites (11_{b}) Ende besitzt, und besagter Hauptkörper (10) und der besagte wenigstens eine selbstwickelnde Streifen (11)
sind zueinander so konfiguriert, dass sie beide sich bewegen können, und zwar aus einer ersten Position, in welcher das besagte erste (10ₐ) und zweite (10_{b}) Ende des besagten Hauptkörpers
(10) gegenübereinander angeordnet und koplanar sind, und besagtes erstes (11ₐ)
und zweites (11_{b}) Ende des besagten wenigstens einen selbstwickelnden Streifens (11) sind
gegenübereinander angeordnet und koplanar, hin zu einer zweiten Position, in welcher besagter selbstwickelnder Streifen (11) und besagter Hauptkörper (10) so angeordnet sind, dass sie gewickelt und um sich selbst bzw. um die genannte Person herum aufgerollt sind.
Besagtes selbstwickelndes Sicherheitsgerät (1) ist **dadurch gekennzeichnet, dass** es umfasst:
Wenigstens ein Paar von Aktivierungsgriffen (14), gekoppelt an den wenigstens einen selbstwickelnden Streifen (11), so konfiguriert, dass er den wenigstens einen selbstwickelnden Streifen (11) und somit den Hauptkörper (10) veranlasst, sich von der besagten ersten Position zur besagten zweiten Position zu bewegen.

2. Gerät (1) nach Anspruch 1, **dadurch gekennzeichnet, dass** jeder Aktivierungsgriff (14) von dem gesagten wenigstens einen Paar von Aktivierungsgriffen (14) im Gebrauch einer Zugkraft ausgesetzt ist, wie der Benutzer ausübt, und wodurch sich der besagte Übergang des besagten zumindest einen selbstwickelnden Streifens (11) und des besagten Hauptkörpers (10) von der besagten ersten Position zur besagten zweiten Position vollzieht.

3. Gerät (1) gemäß einem der vorhergehenden Ansprüche, **dadurch gekennzeichnet, dass** jeder Aktivierungsgriff (14) von dem gesagten wenigstens einen Paar von Aktivierungsgriffen (14) mit einem Seil (14') zum Anfassen des besagten Aktivierungsgriffs (14) durch einen Benutzer ausgestattet ist.

4. Gerät (1) nach Anspruch 3, **dadurch gekennzeichnet, dass** besagtes Seil (14') seinerseits ein erstes (14ₐ) und ein zweites (14_{b}) Ende besitzt, das durch Verbindungsmittel (15) an dem wenigstens einen selbstwickelnden Streifen (11) befestigt ist.

5. Gerät (1) gemäß einem der vorhergehenden Ansprüche, **dadurch gekennzeichnet, dass** es außerdem ein erstes (12ₐ) und ein zweites (12_{b}) Befestigungselement besitzt, das jeweils am besagten ersten (10ₐ) und besagten zweiten (10_{b}) Ende des besagten Hauptkörpers (10) befestigt ist, und besagtes erstes (12ₐ) und besagtes zweites (12_{b}) Befestigungselement sind konfiguriert, um sich in der besagten zweiten Position aneinander festzuhaken.

6. Gerät (1) gemäß einem der vorhergehenden Ansprüche, **Dadurch gekennzeichnet, dass** es eine Mehrzahl von Stützhandgriffen (13) besitzt, integral mit dem besagten Hauptkörper (10) befestigt und konfiguriert, um es dem besagten Hauptkörper (10) zu ermöglichen, erfasst und bewegt zu werden, sowohl in der besagten ersten Position als auch in der besagten zweiten Position.

7. Gerät (1) gemäß einem der vorhergehenden,
**dadurch gekennzeichnet, dass** es eine Mehrzahl von selbstwickelnden Streifen (11) besitzt.

8. Gerät (1) gemäß einem der vorhergehenden Ansprüche, **dadurch gekennzeichnet, dass** besagter Hauptkörper (10) eine erste (10') und eine zweite (10") Oberfläche umfasst.

9. Gerät (1) nach Anspruch 8, **dadurch gekennzeichnet, dass** besagte selbstwickelnde Streifen (11) im besagten Hauptkörper (10) angeordnet sind, zwischen der besagten ersten (10') und zweiten (10") Oberfläche des besagten Hauptkörpers (10).

10. Gerät (1) gemäß einem der vorhergehenden Ansprüche, **dadurch gekennzeichnet, dass** besagter Hauptkörper (10) eine Vielzahl von Stützstreifen (16) besitzt, über eine Erstreckung entlang der Y-Achse verfügt und in der Entsprechung des wenigstens einen selbstwickelnden Streifens (11) angeordnet ist.

## Revendications

1. Dispositif de sécurité auto-enveloppant (1) pour la retenue physique d'une personne, comprenant:
un corps principal (10), pourvu d'une première (10ₐ) et d'une deuxième (10_{b}) extrémité et configuré pour entrer en contact avec une personne,
au moins une bande auto-enveloppante (11), couplée audit corps principal (10), pourvue d'une première (11ₐ) et d'une deuxième (11_{b}) extrémité,
ledit corps principal (10) et ladite au moins une bande auto-enveloppante (11) étant configurés l'un par rapport à l'autre de manière à pouvoir se déplacer tous deux d'une première position, dans laquelle lesdites première (10ₐ) et deuxième (10_{b}) extrémité dudit corps principal (10) sont disposées à l'opposé l'une de l'autre et coplanaires et lesdites première (11ₐ) et deuxième (11_{b}) end extrémités de ladite au moins une bande auto-enveloppante (11) sont disposées à l'opposé l'une de l'autre et coplanaires, à une deuxième position, dans laquelle ladite bande auto-enveloppante (11) et ledit corps principal (10) sont disposés enroulés et roulés sur eux-mêmes ou autour de ladite personne,
ledit dispositif de sécurité auto-enveloppant (1) étant **caractérisé en ce qu'**il comprend
au moins une paire de poignées d'activation (14) couplées à ladite au moins une bande auto-enveloppante (11), configurées pour provoquer le déplacement de ladite au moins une bande auto-enveloppante (11) et donc dudit corps principal (10) de ladite première position à ladite deuxième position.

2. Dispositif (1) selon la revendication 1, **caractérisé en ce que** chaque poignée d'activation (14) de ladite au moins une paire de poignées d'activation (14), en cours d'utilisation, est soumise à une force de traction exercée par un utilisateur, provoquant ledit passage de ladite au moins une bande auto-enveloppante (11) et dudit corps principal.

3. Dispositif (1) selon l'une quelconque des revendications précédentes, **caractérisé en ce que** chaque poignée d'activation (14) de ladite au moins une paire de poignées d'activation (14) est munie d'un câble (14') pour la préhension de ladite poignée d'activation (14) par un utilisateur.

4. Dispositif (1) selon la revendication 3, **caractérisé en ce que** ledit câble (14') comprend à son tour une première (14a) et une deuxième (14b) extrémités fixées à ladite au moins une bande auto-enveloppante (11) par des moyens de connexion (15).

5. Dispositif (1) selon l'une quelconque des revendications précédentes, **caractérisé en ce qu'**il comprend en outre un premier (12a) et un deuxième (12b) éléments de fixation disposés respectivement sur ladite première (10a) et ladite deuxième (10_{b}) extrémités dudit corps principal (10), ledit premier (12ₐ) et ledit deuxième (12_{b}) éléments de fixation étant configurés pour s'accrocher l'un à l'autre dans ladite deuxième position.

6. Dispositif (1) selon l'une quelconque des revendications précédentes, **caractérisé en ce qu'**il comprend une pluralité de poignées de support (13) fixées intégralement avec ledit corps principal (10) et configurées pour permettre audit corps principal (10) d'être saisi et déplacé à la fois dans ladite première position et dans ladite deuxième position.

7. Dispositif (1) selon l'une quelconque des revendications précédentes, **caractérisé en ce qu'**il comprend une pluralité de bandes auto-enveloppantes (11).

8. Dispositif (1) selon l'une quelconque des revendications précédentes, **caractérisé en ce que** ledit corps principal (10) comprend une première (10') et une deuxième (10") surface.

9. Dispositif (1) selon la revendication 8, **caractérisé en ce que**
lesdites bandes auto-enveloppantes (11) sont disposées à l'intérieur dudit corps principal (10),
entre ladite première (10') et ladite deuxième (10") surfaces dudit corps principal (10).

10. Dispositif (1) selon l'une quelconque des revendications précédentes, **caractérisé en ce que** ledit corps principal (10) comprend une pluralité de bandes de support (16), ayant un développement le long de la direction de l'axe Y et agencées en correspondance avec au moins une bande auto-enveloppante (11).
